# EUROPÄISCHE PATENTANMELDUNG

(11) **EP 3 510 946 A1**
(43) Veröffentlichungstag der Anmeldung: **17.07.2019**
(21) Anmeldenummer: 18151085.0
(22) Anmeldetag: 10.01.2018
(51) Int. Cl.: A61B 17/132

(54) **ABSCHNÜRVORRICHTUNG FÜR KÖRPERTEILE**

(71) Anmelder: Kimetec GmbH, 71254 Ditzingen (DE)
(72) Erfinder: Die Erfindernennung liegt noch nicht vor
(74) Vertreter: Fleck, Hermann-Josef

(57) **Zusammenfassung**

Die Erfindung bezieht sich auf eine Abschnürvorrichtung für Körperteile mit einem um ein Körperteil legbaren Abschnürband (10) und einer Verschlussanordnung (2), mittels deren das Abschnürband (10) in seinem um ein Körperteil gelegten gespannten Zustand zu einer Spannschlaufe verriegelbar ist. Eine vorteilhafte Verwendung ergibt sich dadurch, dass das Abschnürband (10) mit einem verdickten Andruckmittel (30) versehen ist, welches an dem Abschnürband (10) angebracht oder in dieses integriert ist (Fig. 1).

## Beschreibung

Die Erfindung bezieht sich auf eine Abschnürvorrichtung für Körperteile mit einem um ein Körperteil legbaren Abschnürband und einer Verschlussanordnung, mittels deren das Abschnürband in seinem um ein Körperteil gelegten gespannten Zustand zu einer Spannschlaufe verriegelbar ist.

Eine Abschnürvorrichtung dieser Art ist in der WO 2015/070992 A1 angegeben. Beispielsweise besteht dabei das Abschnürband aus einem dünnen Bandmaterial, insbesondere Vliesmaterial, und weist eine als Klettverschluss ausgebildete Verschlussvorrichtung mit in dem flauschigen Bandmaterial des Abschnürbandes vorhandenen Mikroschlaufen und einem auf dem Abschnürband aufgebrachten Abschnitt mit zum Verschließen in die Schlaufen eingreifenden Hakenelementen auf. In weiterer Ausgestaltung kann die als Venenstauer ausgebildete Abschnürvorrichtung auch mit einem dort näher beschriebenen Spannungsindikator und/oder Gebrauchsindikator versehen sein. Es sind auch verschiedene Verbindungsweisen zum Anbringen eines oder beider Verschlussteile angegeben, wie Kleben, Schweißen, thermoplastische Verfahren oder Vernähen. Das Bandmaterial kann bei hoher Reißfestigkeit sehr dünn und leicht ausgebildet sein und ist sehr flexibel und flauschig und somit angenehm zum Anlegen an dem betreffenden Körperteil. Das Bandmaterial selbst kann auch eine gewisse Elastizität aufweisen.

Weitere ähnliche Abschnürvorrichtungen für Körperteile sind auch in der DE 20 2013 100 313 U1, der DE 20 2013 100 312 U1 und der DE 20 2014 105 480 U1 gezeigt. Aus diesen Druckschriften gehen auch verschiedene Ausgestaltungen für Verschlussvorrichtungen hervor.

Bei einer in der US 2004/0 092 999 A1 ist ein elastisches Abschnürband mit Klettverschluss, der ein oder zwei Hakenaufsätze aufweist, und mit einem erhabenen Druckstück zum Verschließen einer Gefäßöffnung versehen.

Auch die JP 2003 -61995 A zeigt ein Abschnürband mit Verschlussvorrichtung und Druckstück zum Verschluss einer Gefäßöffnung.

Weitere Abschnürvorrichtungen zeigen die US 3 930 506, wobei zum Fixieren einer um das betreffende Körperteil gelegten Spannschlaufe ein Klebeverschluss vorgesehen ist, die US 3 628 536, die US 2012/0071917 A1 und z. B. die US 5,653,728 A.

In der DE 10 2009 025 416 B4 ist ein Stütz- oder Fixiergurt mit zumindest einem flexiblen, unelastischen Abschnitt und Fixiereinrichtungen angegeben.

Der vorliegenden Erfindung liegt die Aufgabe zugrunde, eine Abschnürvorrichtung der eingangs genannten Art bereitzustellen, die bei einfachem Aufbau und einfacher Handhabung weitere Verwendungsmöglichkeiten ergibt.

Diese Aufgabe wird mit den Merkmalen des Anspruchs 1 gelöst. Hierbei ist vorgesehen, dass das Abschnürband mit einem (gegenüber der Banddicke) verdickten Andruckmittel versehen ist, welches an dem Abschnürband angebracht oder in dieses integriert ist.

Mittels des an dem dünnen Abschnürband angeordneten verdickten Andruckmittels lässt sich ein lokaler Druck an betreffender Stelle des Körperteils ausüben, um z. B. einen dosierten Druck auf ein Blutgefäß auszuüben. Beispielsweise kann auf diese Weise eine Einstichstelle bzw. Öffnung des Blutgefäßes verschlossen werden, wobei vermieden werden kann, dass dieses vollständig abgedrückt wird. Mit dieser Ausbildung des Abschnürbandes kann z. B. ein Dialysepatient nach Entfernen einer Nadel vorteilhaft versorgt werden.

Eine vorteilhafte Ausgestaltung der Abschnürvorrichtung besteht dabei darin, dass die Verschlussanordnung mindestens zwei Verschlusseinheiten aus (jeweils mindestens) zwei verschließend miteinander zusammenwirkenden Verschlussteilen aufweist. Mittels der ersten Verschlusseinheit kann z. B. eine Vorfixierung mit genauer Positionierung des Andruckmittels vorgenommen werden, während dann mit der zweiten Verschlusseinheit die um das Körperteil gelegt und dosiert nachgespannte Schlaufe in ihrer endgültigen Spannstellung fixiert werden kann.

Eine für die Herstellung und Handhabung vorteilhafte Ausgestaltung besteht dabei darin, dass eine erste Verschlusseinheit ein erstes Verschlussteil mit Schlaufen und ein zweites Verschlussteil mit Hakenelementen aufweist, die zum Verhaken mit den Schlaufen (insbesondere Mikroschlaufen) ausgebildet sind, und dass das Abschnürband aus flauschigem, reißfestem Material hoher Zugfestigkeit hergestellt ist. Auch die zweite Verschlusseinheit könnte mit derartigen Verschlussteilen eines Klettverschlusses ausgebildet sein. Alternativ können auch z. B. beide Verschlusseinheiten als Klettverschluss oder Klebeverschluss ausgebildet sein.

Eine weitere vorteilhafte Ausführung wird ferner dadurch erhalten, dass eine zweite Verschlusseinheit ein weiteres erstes Verschlussteil mit Klebeelement aufweist, wobei ein weiteres zweites Verschlussteil durch einen Bandabschnitt gebildet ist. Das Klebeelement kann z. B. als mit einer Abziehfolie versehenes Klebeblättchen ausgebildet sein, das auf dem Abschnürband z. B. durch Kleben oder Schweißen befestigt ist. Das Klebeelement gibt nach Abziehen der Schutzfolie eine sichere Fixierung an dem mit ihm direkt in Verbindung gebrachten Bandabschnitt.

Eine solche Klebeverbindung hat auch den Vorteil, dass eine Wiederverwendung der damit versehenen Abschnürvorrichtung praktisch nicht möglich ist, da die Klebeverbindung nach einem Einmalgebrauch verbraucht ist. In Verbindung mit einer als Klettverschluss ausgebildeten ersten Verschlusseinheit ergibt sich der Vorteil, dass die erste Verschlusseinheit mehrmals zur genauen Positionierung geöffnet und geschlossen werden kann.

Eine vorteilhafte Handhabung und Funktion wird dadurch begünstigt, dass das Andruckmittel im Bereich einer Verschlusseinheit angeordnet ist. Das Andruckmittel kann z. B. durch Kleben, Schweißen oder eine Klettverbindung fixiert sein.

Hierbei ist vorteilhaft vorgesehen, dass das Andruckmittel in einem Querschnittbereich des Abschnürbandes gegenüber einem Verschlussteil einem zuzuordnenden Körperteil zugekehrt angeordnet ist.

Weitere vorteilhafte Maßnahmen für die Ausgestaltung der Abschnürvorrichtung bestehen darin, dass das Andruckmittel an dem Abschnürband z. B. durch eine Schweißverbindung oder Klebeverbindung befestigt ist, abnehmbar mittels Klettverbindung oder Klebeverbindung angebracht ist oder in das Abschnürband eingebettet ist.

Für den Aufbau und die Verwendung ergeben sich weitere vorteilhafte Ausgestaltungsmöglichkeiten dadurch, dass das Abschnürband aus einem Vlies, Filz, papierartigen Material, Viskosematerial oder gewebten Bandmaterial besteht. Beispielsweise kann dabei das Abschnürband mehrschichtig mit einer Abdeckfolie auf einer oder beiden Seiten mit darunter befindlicher Klebeschicht aufgebaut sein. Zum Bilden der Klebeverbindung kann die Abdeckfolie an für den Klebeverschluss vorgesehenen Stellen durchgestanzt sein, um die betreffende Klebestelle auf der Unterseite und/oder Oberseite einfach freilegen zu können. Entsprechend kann auch eine Klebestelle zum Fixieren des Andruckmittels gebildet werden.

Bei einer für die Handhabung vorteilhaften Ausbildung ist des Weiteren vorgesehen, dass die zweite Verschlusseinheit von der ersten Verschlusseinheit in Richtung eines Schlaufenendes einer anzulegenden Schlaufe um mindestens 5 cm entfernt ist. Damit kann z. B. eine Doppelschlaufe gebildet werden.

Eine für die Verwendung (beispielsweise mit einem separaten Andruckmittel) weitere vorteilhafte Ausgestaltung wird auch dadurch erhalten, dass die Verschlussanordnung mindestens zwei Verschlusseinheiten aus zwei verschließend miteinander zusammenwirkenden Verschlussteilen aufweist.

Bei letzterer Ausführung der Abschnürvorrichtung kann die Verschlussanordnung der Abschnürvorrichtung im Einzelnen wie vorstehend angegeben ausgebildet sein.

Die Erfindung wird nachfolgend anhand von Ausführungsbeispielen unter Bezugnahme auf die Zeichnungen näher erläutert. Es zeigen:
- Fig. 1: eine Abschnürvorrichtung mit einem Abschnürband und einer Verschlussanordnung in perspektivischer Ansicht und
- Fig. 2: eine Abschnürvorrichtung mit einer Verschlussanordnung und einem Andruckmittel in seitlicher Ansicht (Mitte), in Draufsicht (oben), und Unteransicht (unten).

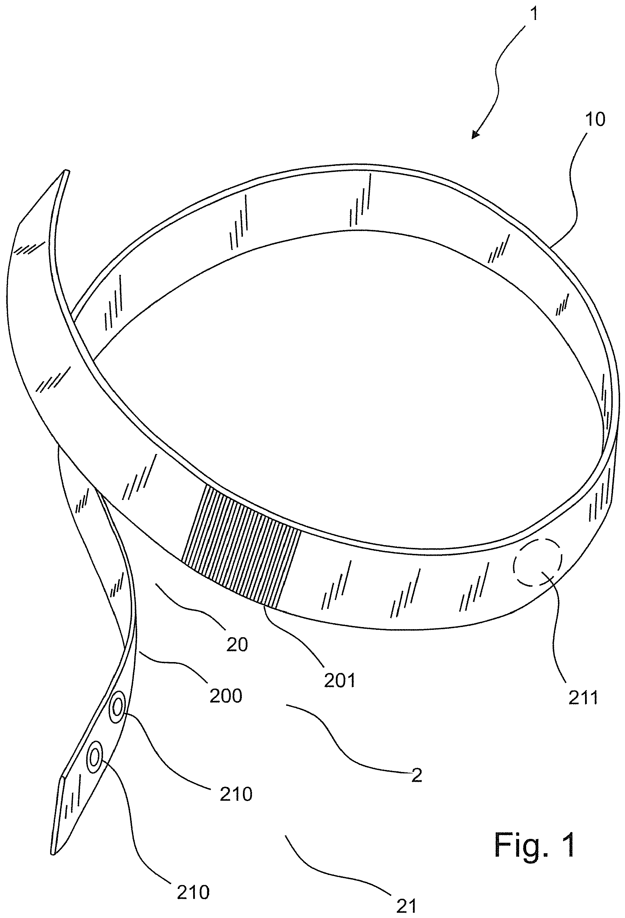

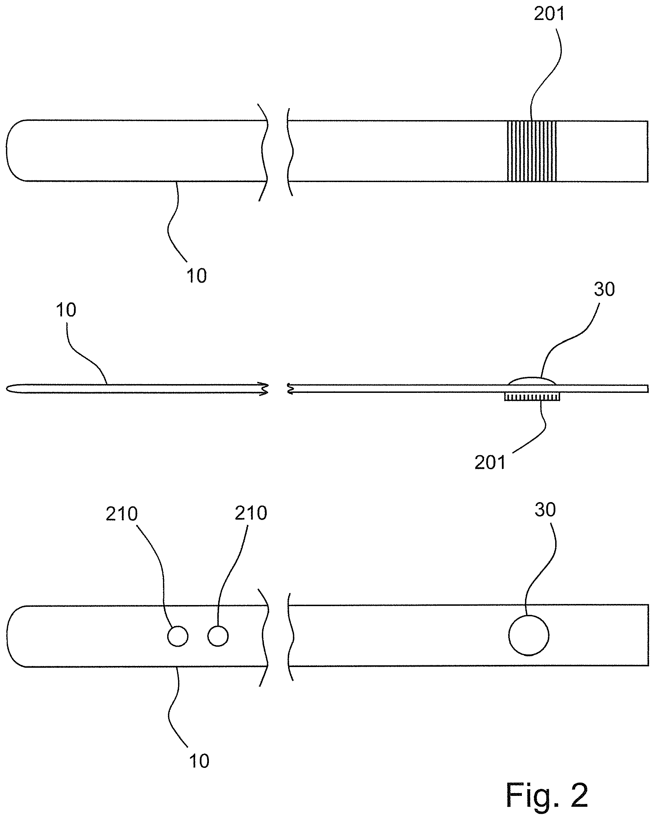

Die in Fig. 1 gezeigte Abschnürvorrichtung 1 weist ein Abschnürband 10 auf, welches mit zwei Verschlusseinheiten, und zwar einer ersten Verschlusseinheit 20 und einer zweiten Verschlusseinheit 21, versehen ist. Die erste Verschlusseinheit 20 weist ein erstes Verschlussteil 200 und ein zweites Verschlussteil 201 auf, während die zweite Verschlusseinheit 21 ein weiteres erstes Verschlussteil 210 und ein weiteres zweites Verschlussteil 211 aufweist. Bei dem gezeigten Ausführungsbespiel ist auf der von dem zweiten Verschlussteil 201 abgekehrten, einem abzuschnürenden Körperteil zuzukehrenden Seite ein (nicht ersichtliches) Andruckmittel angebracht, entsprechend dem in Fig. 2 dargestellten Andruckmittel 30.

Fig. 2 zeigt eine der in Fig. 1 dargestellten Abschnürvorrichtung 1 ähnliche Abschnürvorrichtung in seitlicher Ansicht (mittlere Darstellung), Draufsicht (obere Darstellung) und einer einem abzuschnürenden Körperteil zuzukehrenden Unteransicht (untere Darstellung). In der Seitenansicht und der Unteransicht ist das Andruckmittel 30 zu erkennen, welches gegenüber der unteren Bandfläche deutlich erhaben ist und auf der Unterseite des Abschnürbands 10 angebracht oder in dieses eingebettet ist. Die Fixierung des Andruckmittels 30 an dem Abschnürband 10 kann auf verschiedene Weise vorgenommen werden, beispielsweise durch Kleben, Anschweißen oder eine Klettverbindung oder dergleichen. Die Größe, Form und das Material, insbesondere hinsichtlich Härte, können auf den jeweiligen Behandlungszweck abgestimmt sein, wie z. B. Verschluss eines Blutgefäßes mit mehr oder weniger starkem Andruck, sodass dieses vollständig abgedrückt ist oder aber nur teilweise, wobei ein weiterer Blutfluss durch das Gefäß aufrecht erhalten bleibt. Auch kann das Abschnürband 10 mit dem Andruckmittel 30 für ein nicht geöffnetes Blutgefäß lediglich zum mehr oder weniger starken Abdrücken desselben ausgebildet sein.

Mittels der ersten Verschlusseinheit 20 kann z. B. eine Vorfixierung vorgenommen werden, um das Andruckmittel 30 an der gewünschten Stelle zu positionieren. Dabei ist es für die Bedienung vorteilhaft wenn das Andruckmittel 30 im Querschnittsbereich des zweiten Verschlussteils 201 angeordnet ist, sodass seine Lage auch dann erkennbar ist, wenn es durch das Abschnürband 10 verdeckt und nicht einsehbar ist. Nach der Positionierung und Vorfixierung der mit dem Abschnürband 10 an dem betreffenden Körperteil gebildeten Schlaufe mit dem Andruckmittel 30 kann das Band in dem gewünschten Ausmaß nachgespannt und endgültig mit der zweiten Verschlusseinheit 21 verschlossen werden. Selbstverständlich können bei Bedarf auch mehr als zwei Verschlusseinheiten vorgesehen sein, beispielsweise um eine zusätzliche Verschlussmöglichkeit zu erhalten für den Fall, dass z. B. die zweite Verschlusseinheit 21 verbraucht ist.

Bei einer vorteilhaften Ausgestaltung, wie z. B. bei den in den Fig. 1 und 2 gezeigten Ausführungsbeispielen, ist die erste Verschlusseinheit 20 als Klettverschluss und die zweite Verschlusseinheit 21 als Klebeverschluss ausgebildet. Der erste Verschlussteil 200 wird dabei z. B. durch Schlaufen vorteilhaft durch Schlaufen bzw. Mikroschlaufen des Bandmaterials des Abschnürbands 10 selbst gebildet, während der zweite Verschlussteil 201 durch einen Abschnitt mit in die Schlaufen eingreifenden und an diese angepassten Hakenelementen ausgebildet ist. Der Abschnitt mit den Hakenelementen kann dabei ebenfalls in den Abschnürband 10 integriert sein oder ist vorteilhaft als an dem Abschnürband 10 angebrachter Materialabschnitt mit den Hakenelementen ausgebildet, wie auch in der eingangs genannten Ausgangsdruckschrift WO 2015/070992 A1 ausgeführt, worauf hier zur Vermeidung von Wiederholungen hingewiesen sei.

Die zweite Verschlusseinheit 21 kann z. B. ebenfalls als Klettverschluss ausgebildet sein. Bei dem gezeigten Ausführungsbeispiel ist sie jedoch als Klebeverschluss ausgebildet, wobei ein oder mehrere weitere erste Verschlussteile 210 als Klebeplättchen an dem Abschnürband 10 angebracht sind, während der weitere zweite Verschlussteil 211 durch eine entsprechende Stelle beim Verschließen der Schlaufe durch die Oberfläche des Abschnürbands 10 selbst gebildet wird. Dies ermöglicht eine einfache Fixierung bei entsprechender Schlaufenlänge. Zudem bietet die Ausbildung der zweiten Verschlusseinheit 21 als Klebeverschluss den Vorteil, dass das Abschnürband 10 bzw. die Abschnürvorrichtung 1 praktisch nur einmal verwendbar ist, da nach einem Gebrauch der Klebeverbindung diese weitgehend unbrauchbar wird. Vorteilhaft ist der weitere erste Verschlussteil 210 in Form von Klebeplättchen mit einer abziehbaren Schutzfolie versehen, die erst kurz vor Gebrauch abgezogen wird. Die Klebeplättchen können ihrerseits auf der Unterseite des Abschnürbands 10 angeklebt oder in geeigneter anderer Weise befestigt sein.

Das Abschnürband 10 ist vorteilhaft aus Vlies oder Filz oder papierartigem Material hergestellt und besteht beispielsweise aus Polyester, Polypropylen und/oder Viskosematerial, wie auch in der eingangs genannten Druckschrift WO 2015/070992 A1 ausgeführt ist, worauf diesbezüglich hier ebenfalls ergänzend verwiesen wird. Das Abschnürband 10 kann auch z. B. zwei- oder dreischichtig aufgebaut sein. Dabei kann die mittlere bzw. untere oder obere Schicht mit einer Kleberschicht versehen sein, welche mit einer Abdeckfolie abgedeckt ist. Die Abdeckfolie kann zum teilweisen Freilegen der Kleberschicht an betreffender Stelle durchgestanzt sein.

Das Abschnürband 10 der Abschnürvorrichtung 1 ist vorteilhaft aus einem dünnen, flexiblen und dabei reißfesten Material, insbesondere Spinnvlies, ausgebildet. Durch die sehr flexible Ausbildung des Abschnürbands 1 liegt dieses eng an dem Körperteil an und lässt sich daher gut andrücken und verspannen. Die zu der ersten Verschlusseinheit 20 zusätzlich vorgesehene zweite Verschlusseinheit 21 hat durch die zusätzliche Spannmöglichkeit eine vergleichbare Funktion wie das in der nicht vorveröffentlichten, vorangemeldeten DE 20 2016 106 370 U1 der Anmelderin gezeigte Spannteil, welches eine dosierte zusätzliche Verspannung ermöglicht. Das vorteilhaft verwendete Material des Abschnürbands 10, nämlich dünnes Vliesmaterial, ist z. B. weiterhin vorteilhaft unter Wasserstrahlverfestigung hergestellt, womit eine sehr hohe Reißfestigkeit, bei dünner, dennoch flauschiger Ausbildung erreicht wird. Das so ausgebildete Bandmaterial des Abschnürbands 10 kann z. B. aus flächigem Material in geeignet breite Streifen von z. B. zwischen 0,6 cm und 5 cm geschnitten und als lange, z B. aufgerollte oder gefaltete Bänder zur Verfügung gestellt werden, die mit den beiden Verschlusseinheiten 20, 21 versehen sind, und bedarfsweise abgelängt werden können. Die langen Bänder sind hierzu vorzugsweise in regelmäßigen Abständen mit betreffenden Verschlussteilen 20, 21 versehen, wobei in geeignetem Abstand von den Verschlussteilen Abrissstellen vorgesehen sein können. Die Breite der Abschnürbänder kann sich nach dem Verwendungszweck, beispielsweise zum Abschnüren eines Körperteils eines Erwachsenen oder eines Kindes bzw. Kleinkindes, richten.

Denkbar ist es, bei der gezeigten Abschnürvorrichtung 1 das Andruckmittel 30 wegzulassen und die mindestens zwei Verschlusseinheiten 20, 21 wie beschreiben vorzusehen.

## Patentansprüche

1. Abschnürvorrichtung für Körperteile mit einem um ein Körperteil legbaren Abschnürband (10) und einer Verschlussanordnung (2), mittels deren das Abschnürband (10) in seinem um ein Körperteil gelegten gespannten Zustand zu einer Spannschlaufe verriegelbar ist,
**dadurch gekennzeichnet,**
**dass** das Abschnürband (10) mit einem verdickten Andruckmittel (30) versehen ist, welches an dem Abschnürband (10) angebracht oder in dieses integriert ist.

2. Abschnürvorrichtung nach einem der vorhergehenden Ansprüche,
**dadurch gekennzeichnet,**
**dass** die Verschlussanordnung (2) mindestens zwei Verschlusseinheiten (20, 21) aus zwei verschließend miteinander zusammenwirkenden Verschlussteilen (200, 201; 210, 211) aufweist.

3. Abschnürvorrichtung nach Anspruch 2,
**dadurch gekennzeichnet,**
**dass** eine erste Verschlusseinheit (20) ein erstes Verschlussteil (200) mit Schlaufen und ein zweites Verschlussteil (201) mit Hakenelementen aufweist, die zum Verhaken mit den Schlaufen ausgebildet sind, und
**dass** das Abschnürband (10) aus flauschigem, reißfestem Material hoher Zugfestigkeit hergestellt ist, oder
**dass** beide Verschlusseinheiten (20, 21) als Klettverschluss oder Klebeverschluss ausgebildet sind.

4. Abschnürvorrichtung nach Anspruch 2 oder 3,
**dadurch gekennzeichnet,**
**dass** eine zweite Verschlusseinheit (21) ein weiteres erstes Verschlussteil (210) mit Klebeelement aufweist, wobei ein weiteres zweites Verschlussteil (211) durch einen Bandabschnitt gebildet ist.

5. Abschnürvorrichtung nach einem der vorhergehenden Ansprüche,
**dadurch gekennzeichnet,**
**dass** das Andruckmittel (30) im Bereich einer Verschlusseinheit (20, 21) angeordnet ist.

6. Abschnürvorrichtung nach Anspruch 5,
**dadurch gekennzeichnet,**
**dass** das Andruckmittel (30) in einem Querschnittbereich des Abschnürbandes (10) gegenüber einem Verschlussteil einem zuzuordnenden Körperteil zugekehrt angeordnet ist.

7. Abschnürvorrichtung nach einem der vorhergehenden Ansprüche,
**dadurch gekennzeichnet,**
**dass** das Andruckmittel (30) an dem Abschnürband (10) z. B. durch eine Schweißverbindung oder Klebeverbindung befestigt ist, abnehmbar mittels Klettverbindung oder Klebeverbindung angebracht ist oder in das Abschnürband (10) eingebettet ist.

8. Abschnürvorrichtung nach einem der vorhergehenden Ansprüche,
**dadurch gekennzeichnet,**
**dass** das Abschnürband (10) aus einem Vlies, Filz, papierartigen Material, Viskosematerial oder gewebten Bandmaterial besteht.

9. Abschnürvorrichtung nach einem der vorhergehenden Ansprüche,
**dadurch gekennzeichnet,**
**dass** die zweite Verschlusseinheit (21) von der ersten Verschlusseinheit (20) in Richtung eines Schlaufenendes einer anzulegenden Schlaufe um mindestens 5 cm entfernt ist.

10. Abschnürvorrichtung für Körperteile mit einem um ein Körperteil legbaren Abschnürband (10) und einer Verschlussanordnung (2), mittels deren das Abschnürband (10) in seinem um ein Körperteil gelegten gespannten Zustand zu einer Spannschlaufe verriegelbar ist, insbesondere nach einem der vorhergehenden Ansprüche,
**dadurch gekennzeichnet,**
**dass** die Verschlussanordnung (2) mindestens zwei unterschiedliche Verschlusseinheiten (20, 21) aus zwei verschließend miteinander zusammenwirkenden Verschlussteilen (200, 201; 210, 211) aufweist.
